# FASCICULE DE BREVET EUROPEEN

(11) **EP 3 713 774 B1**
(45) Date de publication et mention de la délivrance du brevet: **09.03.2022**
(21) Numéro de dépôt: 18827181.1
(22) Date de dépôt: 20.11.2018
(51) Int. Cl.: B60C 1/00, C08L 9/06

(54) **COMPOSITION DE CAOUTCHOUC**
KAUTSCHUKZUSAMMENSETZUNG
RUBBER COMPOSITION

(30) Priorité: 21.11.2017 FR 1760959
(43) Date de publication de la demande: 30.09.2020
(73) Titulaire: Compagnie Générale des Etablissements Michelin, 63000 Clermont-Ferrand (FR)
(72) Inventeur: SALIT, Anne-Frédérique, 63040 Clermont-Ferrand Cedex 9 (FR); SCHNELL, Benoît, 63040 Clermont-Ferrand Cedex 9 (FR); GANDER, Sophie, 63040 Clermont-Ferrand Cedex 9 (FR); IVANOV, Sergey, 63040 Clermont-Ferrand Cedex 9 (FR); FLEURY, Etienne, 63040 Clermont-Ferrand Cedex 9 (FR)
(74) Mandataire: Gandon-Pain, Sylvie
(86) Numéro de dépôt international: PCT/FR2018/052916
(87) Numéro de publication internationale: WO 2019/102131

(56) Documents cités:
- WO-A1-2017/009150
- US-A1- 2012 046 418
- US-A1- 2013 123 418

## Description

Le domaine de la présente invention est celui des compositions de caoutchouc diénique renforcées par une charge et utilisables notamment pour la fabrication de pneumatiques pour véhicules.

Il est connu d'introduire des composés 1,3-dipolaires dans des compositions de caoutchouc diénique au moment de la préparation de ces dernières. On peut par exemple se référer à la demande de brevet WO 2012007442. Il y est décrit que les composés 1,3-dipolaires réagissent typiquement avec les élastomères diéniques de la composition de caoutchouc. La réactivité du dipôle du composé 1,3-dipolaire vis-à-vis des élastomères diéniques permet de greffer des groupes fonctionnels sur les élastomères diéniques de la composition de caoutchouc

Il est également connu d'utiliser dans des compositions de caoutchouc des élastomères diéniques modifiés en ce qu'ils portent des groupes pendants glycidyle. De tels élastomères sont par exemple décrits dans la demande de brevet US 2012/0046418 A1 et sont obtenus par la réaction d'un composé 1,3-dipolaire portant un groupe glycidyle. Mais il s'avère que les élastomères ainsi modifiés confèrent à la composition de caoutchouc qui les contient des propriétés à la rupture dégradées.

Or, une composition de caoutchouc réticulée doit présenter de bonnes propriétés à la rupture pour pouvoir être utilisée dans un article semi-fini pour pneumatique. En effet, le pneumatique au cours du roulage est soumis à de fortes contraintes et à de grandes déformations, sachant qu'il doit aussi présenter une résistance au roulement la plus faible possible.

La Demanderesse a découvert, de façon surprenante, qu'une composition de caoutchouc comprenant un composé 1,3-dipolaire portant un groupe époxyde substitué particulier présente des propriétés à la rupture et une mise en œuvre améliorées ainsi que de bonnes propriétés d'hystérèse.

Un premier objet de l'invention est une composition de caoutchouc à base d'au moins un élastomère diénique, une charge renforçante et un composé 1,3-dipolaire comportant un groupe époxyde, le groupe époxyde étant un cycle éther à 3 membres dans lequel un premier membre est un atome de carbone présentant un rattachement au dipôle du composé 1,3-dipolaire et un deuxième membre est un carbone tertiaire ou quaternaire.

Un autre objet de l'invention est un pneumatique qui comprend une composition de caoutchouc conforme à l'invention.

L'invention concerne également un procédé pour fabriquer une composition de caoutchouc conforme à l'invention.

### I. DESCRIPTION DETAILLEE DE L'INVENTION

Dans la présente description, sauf indication expresse différente, tous les pourcentages (%) indiqués sont des % en masse. L'abréviation "pce" signifie parties en poids pour cent parties d'élastomère (du total des élastomères si plusieurs élastomères sont présents).

D'autre part, tout intervalle de valeurs désigné par l'expression "entre a et b" représente le domaine de valeurs supérieur à "a" et inférieur à "b" (c'est-à-dire bornes a et b exclues) tandis que tout intervalle de valeurs désigné par l'expression "de a à b" signifie le domaine de valeurs allant de "a" jusqu'à "b" (c'est-à-dire incluant les bornes strictes a et b).

Par l'expression composition "à base de", il faut entendre une composition comportant le mélange et/ou le produit de réaction des différents constituants utilisés, certains de ces constituants de base étant susceptibles de, ou destinés à, réagir entre eux, au moins en partie, lors des différentes phases de fabrication de la composition, en particulier au cours de sa réticulation ou vulcanisation.

Les composés mentionnés dans la description peuvent être d'origine fossile ou biosourcés. Dans ce dernier cas, ils peuvent être, partiellement ou totalement, issus de la biomasse ou obtenus à partir de matières premières renouvelables issues de la biomasse. Sont aussi concernés les élastomères, les plastifiants, les charges....

Par élastomère diénique, doit être compris un élastomère comprenant des unités monomères diéniques, en particulier des unités de monomères 1,3-diènes. On entend par unité monomère diénique toute unité qui résulte de l'insertion d'un diène dans une chaîne polymère et qui contient une double liaison carbone carbone.

Le terme composé 1,3-dipolaire est compris selon la définition donnée par IUPAC. Par définition, il contient un dipôle.

Dans la présente demande, on entend par groupe carboné un groupe qui contient un ou plusieurs atomes de carbone. On entend aussi par groupe d'atomes un groupe constitué par l'enchaînement de plusieurs atomes liés de façon covalente.

Dans la présente demande, un atome de carbone qui est membre du cycle époxyde est qualifié de carbone tertiaire lorsque l'atome de carbone est relié directement de façon covalente à deux atomes de carbone dont un est aussi membre du cycle époxyde et à l'atome d'oxygène membre du cycle époxyde. Un atome de carbone qui est membre du cycle époxyde est qualifié de carbone quaternaire lorsque l'atome de carbone est relié directement de façon covalente à trois atomes de carbone dont un est aussi membre du cycle époxyde et à l'atome d'oxygène membre du cycle époxyde.

De manière connue, un groupe époxyde est un cycle éther à 3 membres dont 2 membres sont deux atomes de carbone et le troisième membre est un atome d'oxygène. Le groupe époxyde dans le composé 1,3-dipolaire utile aux besoins de l'invention est un cycle éther à 3 membres dont un premier membre est un atome de carbone présentant un rattachement au dipôle du composé 1,3-dipolaire et un deuxième membre est un carbone tertiaire ou quaternaire. De préférence, le groupe époxyde est de formule (I).

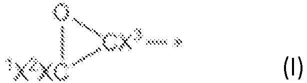

Dans la formule (I), le symbole ^{∗} représente un rattachement au dipôle ; les symboles X¹, X² et X³, identiques ou différents, représentent un atome d'hydrogène ou un groupe substituant, et au moins un des symboles X¹, X² et X³ est différent d'un atome d'hydrogène.

Selon un mode de réalisation préférentiel, X³ représente un atome d'hydrogène, ce qui revient à dire que le premier membre du cycle époxyde est un carbone tertiaire.

Selon un autre mode de réalisation préférentiel, X¹ représente un groupe substituant et X² représente un atome d'hydrogène, auquel cas le deuxième membre du cycle époxyde est un carbone tertiaire.

Selon un autre mode de réalisation préférentiel de l'invention, X¹ et X² représentent chacun un groupe substituant, auquel cas le deuxième membre du cycle époxyde est un carbone quaternaire.

De préférence, le groupe substituant, représenté par les symboles X¹ X² ou X³, est un groupe carboné, en particulier hydrocarboné. Le groupe substituant peut être aliphatique ou aromatique, linéaire, ramifié ou cyclique. Comme groupe substituant conviennent particulièrement les alkyles et les aryles, plus particulièrement les alkyles ayant 1 à 6 atomes de carbone, de préférence méthyle, ou les aryles ayant 6 à 12 atomes de carbone, de préférence phényle.

De préférence, le dipôle du composé 1,3-dipolaire est un dipôle contenant au moins un atome d'azote, de manière plus préférentielle un dipôle choisi dans le groupe constitué par le dipôle oxyde de nitrile, le dipôle nitrone et le dipôle nitrile imine. Autrement dit, le composé 1,3-dipolaire est plus préférentiellement choisi dans le groupe constitué par les oxydes de nitrile, les nitrones et les nitriles imine. Avantageusement, le composé 1,3-dipolaire est un oxyde de nitrile, auquel cas le dipôle du composé 1,3-dipolaire est un oxyde de nitrile -C≡N→O.

Selon un mode de réalisation, le composé 1,3-dipolaire comprend un noyau benzénique substitué par le dipôle du composé 1,3-dipolaire et de préférence aussi substitué en ortho du dipôle. Très avantageusement, le composé 1,3 dipolaire est un oxyde de nitrile aromatique, c'est-à-dire un composé aromatique substitué par un dipôle oxyde de nitrile. Mieux, le composé 1,3-dipolaire est un monooxyde de nitrile aromatique, ce qui correspond à un composé qui contient un seul dipôle oxyde de nitrile et qui est un composé aromatique substitué par le dipôle oxyde de nitrile.

Selon un mode de réalisation très particulier de l'invention, le composé 1,3-dipolaire contient un motif de formule (II) dans laquelle quatre des cinq symboles R1 à R5, identiques ou différents, sont chacun un atome ou un groupe d'atomes, et le cinquième symbole représente une chaîne carbonée permettant le rattachement au groupe époxyde, sachant qu'au moins un des R1 et R5 est différent d'un atome d'hydrogène.

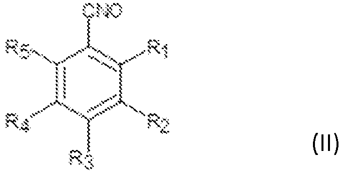

On entend par groupe d'atomes un enchaînement d'atomes liés de façon covalente pour former une chaîne. Deux groupes Ri et Ri+1, pour i nombre entier allant 1 à 4, peuvent former ensemble avec les atomes de carbone du noyau benzénique auxquels ils se rattachent, un cycle.

De préférence R1, R3 et R5 représentent chacun un groupe hydrocarboné et R2 ou R4 représente le cinquième symbole. De manière plus préférentielle R1, R3 et R5 représentent chacun un alkyle, de manière encore plus préférentielle un méthyle ou un éthyle.

La chaine carbonée représentée par le cinquième symbole peut être aliphatique ou aromatique, linéaire, ramifiée ou cyclique, de préférence saturée. Le cinquième symbole représente préférentiellement une chaîne carbonée interrompue par un ou plusieurs hétéroatomes, de préférence oxygène. On entend par chaîne carbonée une chaîne qui comprend un ou plusieurs atomes de carbone. La chaîne carbonée peut être une chaîne hydrocarbonée. La chaîne carbonée peut comprendre une ou plusieurs fonctions éther, en particulier le cinquième symbole comprend un motif -CH₂O-, le groupe méthylène étant attaché au groupe époxyde.

Très avantageusement, le composé 1,3-dipolaire est un composé de formule (III), (IV) ou (V).

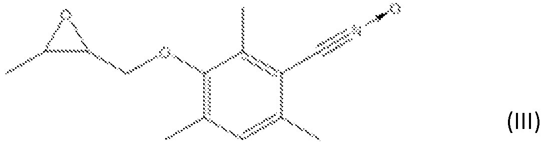

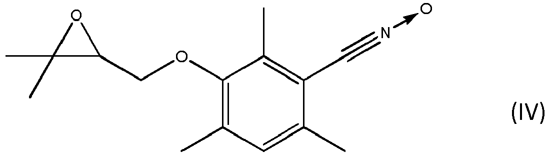

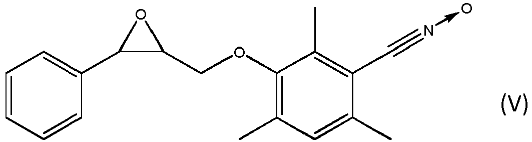

Le taux de composé 1,3-dipolaire peut varier dans une large mesure selon l'application envisagée de la composition de caoutchouc. Selon l'un quelconque des modes de réalisation de l'invention, le composé 1,3-dipolaire est préférentiellement introduit dans la composition de caoutchouc à un taux allant de 0,01 à 5% molaire, plus préférentiellement de 0,01 à 1% molaire, encore plus préférentiellement de 0,1 à 1% molaire. Ce taux exprimé en pourcentage molaire, équivaut au nombre de moles de composé 1,3-dipolaire pour 100 moles d'unités monomères diéniques du polymère diénique utile aux besoins de l'invention.

L'élastomère diénique utile aux besoins de l'invention peut être :
(a) - tout homopolymère d'un monomère diène conjugué, notamment tout homopolymère obtenu par polymérisation d'un monomère diène conjugué ayant de 4 à 12 atomes de carbone ;
(b) - tout copolymère obtenu par copolymérisation d'un ou plusieurs diènes conjugués entre eux ou avec un ou plusieurs composés vinyle aromatique ayant de 8 à 20 atomes de carbone ;
(c) - un copolymère ternaire obtenu par copolymérisation d'éthylène, d'une α-oléfine ayant 3 à 6 atomes de carbone avec un monomère diène non conjugué ayant de 6 à 12 atomes de carbone, comme par exemple les élastomères obtenus à partir d'éthylène, de propylène avec un monomère diène non conjugué du type précité tel que notamment l'hexadiène-1,4, l'éthylidène norbornène, le dicyclopentadiène ;
(d) - un copolymère d'isobutène et d'isoprène (caoutchouc butyle), ainsi que les versions halogénées, en particulier chlorées ou bromées, de ce type de copolymère ;
(e) - tout copolymère obtenu par copolymérisation d'un ou plusieurs diènes conjugués avec l'éthylène, une α-monooléfine aliphatique acyclique ayant 3 à 18 atomes de carbone ou leur mélange comme par exemple ceux décrits dans le document WO 2005028526, WO 2004035639 et WO 2007054224.

De préférence, l'élastomère diénique est choisi dans le groupe des élastomères constitué par les homopolymères de 1,3-butadiène, les homopolymères d'isoprène, les copolymères de 1,3-butadiène, les copolymères d'isoprène et leurs mélanges.

La composition de caoutchouc comporte tout type de charge dite renforçante, connue pour ses capacités à renforcer une composition de caoutchouc utilisable pour la fabrication de pneumatiques, par exemple une charge organique telle que du noir de carbone, une charge inorganique renforçante telle que de la silice à laquelle est associé de manière connue un agent de couplage, ou encore un mélange de ces deux types de charge. Une telle charge renforçante consiste typiquement en des nanoparticules dont la taille moyenne (en masse) est inférieure au micromètre, généralement inférieure à 500 nm, le plus souvent comprise entre 20 et 200 nm, en particulier et plus préférentiellement comprise entre 20 et 150 nm.

Selon un mode de réalisation particulier de l'invention, la charge renforçante comprend une charge inorganique renforçante, préférentiellement une silice. Selon ce mode de réalisation, la charge inorganique renforçante représente plus de 50% en masse de la masse de la charge renforçante de la composition de caoutchouc. On dit alors que la charge inorganique renforçante est majoritaire.

Lorsqu'il est combiné à une charge inorganique renforçante majoritaire telle que la silice, le noir de carbone est utilisé de préférence à un taux inférieur à 20 pce, plus préférentiellement inférieur à 10 pce (par exemple entre 0.5 et 20 pce, notamment entre 2 et 10 pce). Dans les intervalles indiqués, on bénéficie des propriétés colorantes (agent de pigmentation noire) et anti-UV des noirs de carbone, sans pénaliser par ailleurs les performances typiques apportées par la charge inorganique renforçante.

De manière préférentielle, le taux de charge renforçante totale est compris entre 30 et 160 pce, plus préférentiellement entre 40 pce et 160 pce. En deçà de 30 pce, le renforcement de la composition de caoutchouc est insuffisant pour apporter un niveau de cohésion ou de résistance à l'usure adéquats du composant caoutchouteux du pneumatique comprenant cette composition. De manière encore plus préférentielle, le taux de charge renforçante totale est d'au moins 50 pce. Au-delà de 160 pce, il existe un risque d'augmentation de l'hystérèse et donc de la résistance au roulement des pneumatiques. Pour cette raison, le taux de charge renforçante totale est de préférence dans un domaine allant de 50 à 120 pce, notamment pour un usage dans une bande de roulement de pneumatique. L'une quelconque de ces plages de taux de charge renforçante totale peut s'appliquer à l'un quelconque des modes de réalisation de l'invention.

Pour coupler la charge inorganique renforçante à l'élastomère diénique, on utilise de manière bien connue un agent de couplage, notamment un silane, (ou agent de liaison) au moins bifonctionnel destiné à assurer une connexion suffisante, de nature chimique et/ou physique, entre la charge inorganique (surface de ses particules) et l'élastomère diénique. On utilise en particulier des organosilanes ou des polyorganosiloxanes au moins bifonctionnels. Plus particulièrement, on utilise des silanes polysulfurés, dits "symétriques" ou "asymétriques" selon leur structure particulière, tels que décrits par exemple dans les demandes WO03/002648 (ou US 2005/016651) et WO03/002649 (ou US 2005/016650). A titre d'exemples de silanes polysulfurés, on citera plus particulièrement les polysulfures (notamment disulfures, trisulfures ou tétrasulfures) de bis-(alkoxyl(C₁-C₄)-alkyl(C₁-C₄)silyl-alkyl(C₁-C₄)), comme par exemple les polysulfures de bis(3-triméthoxysilylpropyl) ou de bis(3-triéthoxysilylpropyl). Parmi ces composés, on utilise en particulier le tétrasulfure de bis(3-triéthoxysilylpropyl), en abrégé TESPT, de formule [(C₂H₅O)₃Si(CH₂)₃S₂]₂ ou le disulfure de bis-(triéthoxysilylpropyle), en abrégé TESPD, de formule [(C₂H₅O)₃Si(CH₂)₃S]₂.

La teneur en agent de couplage est avantageusement inférieure à 20 pce, étant entendu qu'il est en général souhaitable d'en utiliser le moins possible. Typiquement le taux d'agent de couplage représente de 0,5% à 15% en poids par rapport à la quantité de charge inorganique. Son taux est préférentiellement compris entre 0,5 et 12 pce, plus préférentiellement compris dans un domaine allant de 3 à 10 pce. Ce taux est aisément ajusté par l'homme du métier selon le taux de charge inorganique utilisé dans la composition.

La composition de caoutchouc conforme à l'invention peut également contenir, en complément des agents de couplage, des activateurs de couplage, des agents de recouvrement des charges inorganiques ou plus généralement des agents d'aide à la mise en œuvre susceptibles de manière connue, grâce à une amélioration de la dispersion de la charge dans la matrice de caoutchouc et à un abaissement de la viscosité des compositions, d'améliorer leur faculté de mise en œuvre à l'état cru.

La composition de caoutchouc conforme à l'invention peut comporter également tout ou partie des additifs usuels habituellement utilisés dans les compositions d'élastomères destinées à constituer des mélanges externes d'articles finis en caoutchouc tels que des pneumatiques, en particulier de bandes de roulement, comme par exemple des plastifiants ou des huiles d'extension, des pigments, des agents de protection tels que cires anti-ozone, anti-ozonants chimiques, anti-oxydants, des agents anti-fatigue, des résines renforçantes (tels que résorcinol ou bismaléimide), des accepteurs (par exemple résine phénolique novolaque) ou des donneurs de méthylène (par exemple HMT ou H3M) tels que décrits par exemple dans la demande WO 02/10269, un système de réticulation, des accélérateurs ou retardateurs de vulcanisation, des activateurs de vulcanisation.

La composition de caoutchouc comprend de préférence un système de réticulation, ce qui permet d'améliorer ses propriétés élastiques. Le système de réticulation est de préférence à base de soufre (ou d'un agent donneur de soufre) et d'un accélérateur primaire de vulcanisation, c'est-à-dire un système de vulcanisation A ce système de vulcanisation de base viennent s'ajouter, incorporés au cours de la première phase non-productive et/ou au cours de la phase productive telles que décrites ultérieurement, divers accélérateurs secondaires ou activateurs de vulcanisation connus tels qu'oxyde de zinc, acide stéarique ou composés équivalents, dérivés guanidiques (en particulier diphénylguanidine), ou encore des retardateurs de vulcanisation connus. Le soufre est utilisé à un taux préférentiel compris entre 0,5 et 12 pce, en particulier entre 1 et 10 pce. L'accélérateur primaire de vulcanisation est utilisé à un taux préférentiel compris entre 0,5 et 10 pce, plus préférentiellement compris entre 0,5 et 5,0 pce. Le système de réticulation peut être à base de polyacides, notamment diacides comme décrits dans les demandes de brevet WO2014095582 et WO2014095585.

La composition de caoutchouc conforme à l'invention est fabriquée dans des mélangeurs appropriés, en utilisant deux phases de préparation successives bien connues de l'homme du métier : une première phase de travail ou malaxage thermomécanique (phase dite « non-productive ») à haute température, jusqu'à une température maximale comprise entre 130°C et 200°C, suivie d'une seconde phase de travail mécanique (phase dite « productive ») jusqu'à une plus basse température, typiquement inférieure à 110°C, par exemple entre 40°C et 100°C, phase de finition au cours de laquelle est incorporé le système de réticulation.

Selon un mode de réalisation particulier de l'invention, la composition de caoutchouc conforme à l'invention peut être fabriquée par un procédé, autre objet de l'invention, qui comprend les étapes suivantes :
- au cours d'une première étape dite non productive malaxer l'élastomère diénique, le composé 1,3-dipolaire tel que défini précédemment en malaxant thermo-mécaniquement,
- ajouter ensuite la charge renforçante, le cas échéant les autres ingrédients de la composition de caoutchouc à l'exception du système de réticulation en malaxant thermo-mécaniquement jusqu'à atteindre une température maximale comprise entre 130 et 200°C,
- refroidir l'ensemble à une température inférieure à 100°C,
- incorporer ensuite le système de réticulation,
- malaxer le tout jusqu'à une température maximale inférieure à 120°C.

Le temps de contact entre l'élastomère diénique et le composé 1,3-dipolaire qui sont malaxés thermo-mécaniquement est ajusté en fonction des conditions du malaxage thermomécanique, notamment en fonction de la température. Plus la température du malaxage est élevée, plus ce temps de contact est court. Typiquement il est de 1 à 5 minutes pour une température de 100 à 130°C.

Après l'incorporation de tous les ingrédients de la composition de caoutchouc, la composition finale ainsi obtenue est ensuite calandrée, par exemple sous la forme d'une feuille ou d'une plaque, notamment pour une caractérisation au laboratoire, ou encore extrudée, pour former par exemple un profilé de caoutchouc utilisé comme composant caoutchouteux pour la confection du pneumatique, notamment une bande de roulement pour pneumatique.

La composition de caoutchouc conforme à l'invention peut être soit à l'état cru (avant réticulation ou vulcanisation), soit à l'état cuit (après réticulation ou vulcanisation). Elle est préférentiellement utilisée dans un pneumatique, par exemple comme article semi-fini.

Les caractéristiques précitées de la présente invention, ainsi que d'autres, seront mieux comprises à la lecture de la description suivante de plusieurs exemples de réalisation de l'invention, donnés à titre illustratif et non limitatif.

### II. EXEMPLES DE REALISATION DE L'INVENTION

### II.1-Mesures et tests utilisés :

### Analyse RMN :

L'analyse structurale ainsi que la détermination des puretés molaires des molécules de synthèses sont réalisées par une analyse RMN. Les spectres sont acquis sur un spectromètre Avance 3 400 MHz BRUKER équipé d'une sonde " large bande " BBFO-zgrad 5 mm. L'expérience RMN ¹H quantitative, utilise une séquence simple impulsion 30° et un délai de répétition de 3 secondes entre chacune des 64 acquisitions. Les échantillons sont solubilisés dans le Diméthylsulfoxide deutéré (DMSO). Ce solvant est également utilisé pour le signal de lock. La calibration est réalisée sur le signal des protons du DMSO deutéré à 2.44ppm par rapport à une référence TMS à 0ppm. Le spectre RMN ¹H couplé aux expériences 2D HSQC ¹H/13C et HMBC ¹H/¹³C permettent la détermination structurale des molécules (cf tableaux d'attributions). Les quantifications molaires sont réalisées à partir du spectre RMN 1D ¹H quantitatif.

La détermination du taux molaire de composé oxyde de nitrile greffé est réalisée par une analyse RMN. Les spectres sont acquis sur un spectromètre 500 MHz BRUKER équipé d'une « CryoSonde BBFO-zgrad-5 mm ». L'expérience RMN ¹H quantitative, utilise une séquence simple impulsion 30° et un délai de répétition de 5 secondes entre chaque acquisition. Les échantillons sont solubilisés dans le chloroforme deutéré (CDCl3) dans le but d'obtenir un signal de « lock ».

Des expériences RMN 2D ont permis de vérifier la nature du motif greffé grâce aux déplacements chimiques des atomes de carbone et de proton.

### Essais de traction :

Les allongements à la rupture et les contraintes à la rupture sont mesurés par des essais de traction selon la norme française NF T 46-002 de septembre 1988. Toutes ces mesures de traction sont effectuées dans les conditions normales de température (23 ± 30 2°C) et d'hygrométrie (50 ± 5% d'humidité relative), selon la norme française NF T 40-101 (décembre 1979).

### Propriétés dynamiques :

Les propriétés dynamiques tan(δ)max sont mesurées sur un viscoanalyseur (Metravib VA4000), selon la norme ASTM D 5992-96. On enregistre la réponse d'un échantillon de composition vulcanisée (éprouvette cylindrique de 4 mm d'épaisseur et de 400 mm² de section), soumis à une sollicitation sinusoïdale en cisaillement simple alterné, à la fréquence de 10Hz, dans les conditions normales de température (23°C) selon la norme ASTM D 1349-99. On effectue un balayage en amplitude de déformation de 0,1% à 100% (cycle aller), puis de 100% à 0,1% (cycle retour). Les résultats exploités sont le module complexe de cisaillement dynamique (G^{∗}) à 25% de déformation, le facteur de perte tan(δ) et l'écart de module (ΔG^{∗}) entre les valeurs à 0,1 et 100% de déformation (effet Payne). Pour le cycle retour, on indique la valeur maximale de tan(δ) observée, noté tan(δ)max.

### Rhéometrie :

Les mesures sont effectuées à 150°C avec un rhéomètre à chambre oscillante, selon la norme DIN 53529 - partie 3 (juin 1983). Les mesures sont traitées selon la norme DIN 53529 - partie 2 (mars 1983). L'évolution du couple rhéométrique en fonction du temps décrit l'évolution de la rigidification de la composition par suite de la réaction de vulcanisation et permet ainsi de suivre l'avancement de la vulcanisation. On mesure pour chaque composition la valeur de couple minimum Cmin. Le Cmin est représentatif de la viscosité à cru (avant vulcanisation) de la composition de caoutchouc et permet d'appréhender l'aptitude de la composition de caoutchouc à être mise en œuvre (en anglais « processability »).

### II.2-Synthèse des composés 1,3-dipolaires :

Les composés 1,3-dipolaires suivants ont été préparés, respectivement D-1, D-2, D-3 et D-4.

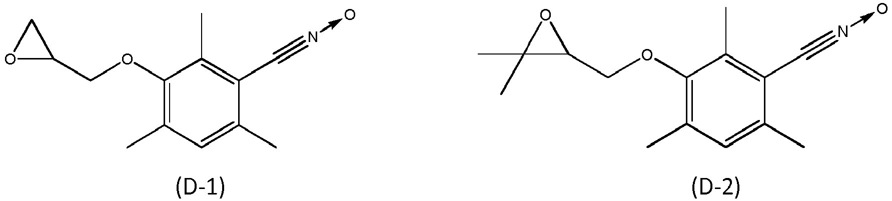

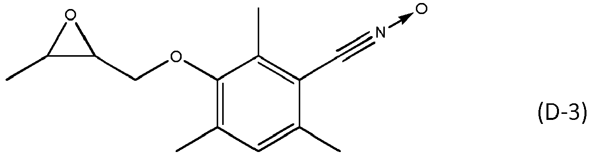

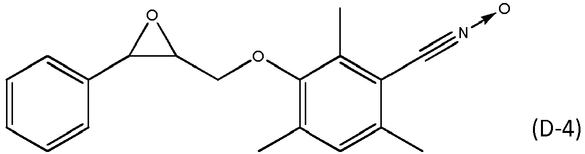

### Synthèse du 3-hydroxy-2,4,6-triméthylbenzaldéhyde (cible 1) :

Le composé cible 1 (ou A) est un précurseur commun utilisé dans la synthèse de certains des composés 1,3-dipolaires. Il est synthétisé selon le schéma suivant :

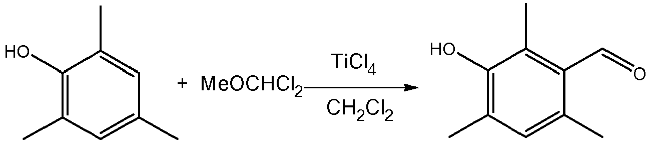

Le composé cible 1 peut être obtenu selon une procédure décrite dans l'article Yakubov, A. P.; Tsyganov, D. V.; Belen'kii, L. I.; Krayushkin, M. M. Bulletin of the Academy of Sciences of the USSR, Division of Chemical Science (English Translation); vol. 40; nb. 7.2; (1991); p. 1427 -1432*;* Izvestiya Akademii Nauk SSSR, Seriya Khimicheskaya; nb. 7; (1991); p. 1609 -1615*.*

### Synthèse du 2,4,6-triméthyl-3-(oxiran-2-ylméthoxy)benzonitrile oxyde (D-1) :

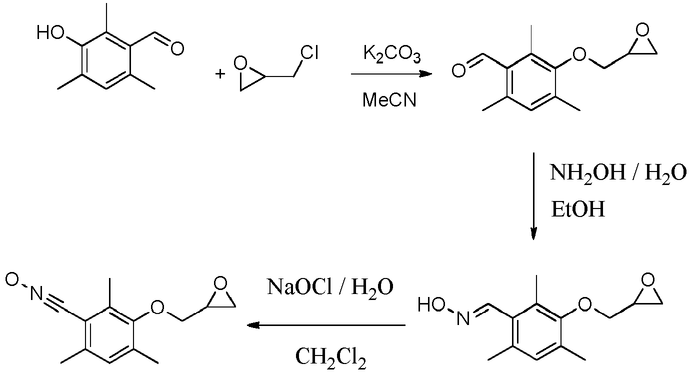

### Synthèse de 2,4,6-triméthyl-3-(oxiran-2-ylméthoxy)benzaldéhyde (cible 2) :

A un mélange de 3-hydroxy-2,4,6-triméthylbenzaldéhyde (40,00 g, 0,244 mol) et d'épichlorhydrine (56,35 g, 0,609 mol) dans l'acétonitrile (100 ml) est ajouté le carbonate de potassium (50,50 g, 0,365 mol). Le milieu réactionnel est agité pendant 3 heures à 60 ºC et ensuite agité pendant 2.5-3 heures à 70 ºC. Après retour à 40-50 °C, le mélange réactionnel est dilué avec un mélange d'eau (250ml) et d'éthylacétate (250 ml), puis maintenu sous agitation pendant 10 minutes. La phase organique est séparée et lavée avec de l'eau (4 fois par 125 ml). Le solvant est évaporé sous pression réduite (T_{bain} 37 ºC, 40 mbar). Une huile rouge (66,43 g) est obtenue.

Le produit secondaire de la réaction, le 3,3'-((2-hydroxypropane-1,3-diyl)bis(oxy))bis(2,4,6-triméthylbenzaldéhyde) est séparé du produit cible 2 par chromatographie sur colonne de silice (éluent : éthylacétate / éther de pétrole = 1 / 4). Après récupération des fractions du produit cible 2, les solvants sont évaporés sous pression réduite (Tbain 36 ºC, 21 mbar). De l'éther de pétrole (120 ml) est ajouté au résidu et la suspension est maintenue sous agitation à -18ºC pendant 2 heures. Le précipité est filtré et lavé sur le filtre avec de l'éther de pétrole (40/60) (3 fois 25 ml) et enfin séché pendant 10-15 heures sous pression atmosphérique à température ambiante. Un solide blanc (40,04 g, rendement massique de 75 %) avec un point de fusion de 52 °C est obtenu. La pureté molaire est supérieure à 99 % (RMN ¹H).

**Tableau d'attribution :**

| 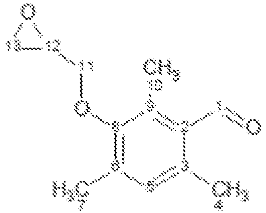 | | |
|---|---|---|
| | δ ¹H (ppm) | δ ¹³C (ppm) |
| 1 | 10.37 | 193.3 |
| 2 | / | 131.1 |
| 3 | / | 132.8 |
| 4 | 2.4 | 19.2 |
| 5 | 6.94 | 131.3 |
| 6 | / | 136.3 |
| 7 | 2.2 | 16.1 |
| 8 | / | 153.4 |
| 9 | / | 135.7 |
| 10 | 2.4 | 11.7 |
| 11 | 3.50/4.00 | 73.4 |
| 12 | 3.29 | 49.6 |
| 13 | 2.60/2.76 | 42.9 |

### Solvant DMSO

### Synthèse de 2,4,6-triméthyl-3-(oxiran-2-ylmethoxy)benzaldéhyde oxime (cible 3) :

A une solution de 2,4,6-triméthyl-3-(oxiran-2-ylméthoxy)benzaldéhyde (46,70 g, 0,212 mol) dans l'alcool éthylique (750 ml) est ajoutée à température ambiante une solution d'hydroxylamine (16,81 g, 0,254 mol, 50 % dans l'eau, Aldrich) dans l'alcool éthylique (75 ml). Le milieu réactionnel est agité pendant 3 heures à 23°C (T_{bain}). Après évaporation du solvant (T_{bain} 24°C, 35 mbar), de l'éther de pétrole (40/60) (150 ml) est ajouté. Le précipité est filtré et lavé sur le filtre par l'éther de pétrole (100 ml). Le produit brut est solubilisé dans un mélange d'acétate d'éthyle (650 ml) et d'éther de pétrole (650 ml) à température ambiante et cette solution est filtrée sur une couche de silicagel (Ø9 cm, 2,0 cm de SiO₂). Les solvants sont évaporés (T_{bain} 22-24°C) et le produit cible 3 est séché sous pression atmosphérique à température ambiante. Un solide blanc (43,81 g, rendement massique de 88 %) avec un point de fusion de 77 °C est obtenu. La pureté molaire est supérieure à 99 % (RMN ¹H)

**Tableau d'attribution :**

| 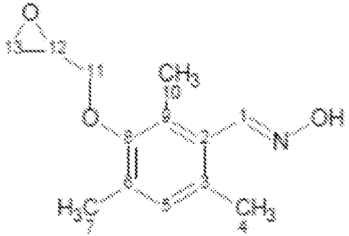 | | |
|---|---|---|
| | δ ¹H (ppm) | δ ¹³C (ppm) |
| 1 | 8.2 | 147.3 |
| 2 | / | 129.1 |
| 3 | / | 129.2 |
| 4 | 2.18 | 20.1 |
| 5 | 6.85 | 130.2 |
| 6 | / | 130.3 |
| 7 | 2.15 | 15.7 |
| 8 | / | 153.1 |
| 9 | / | 131.7 |
| 10 | 2.18 | 13.1 |
| 11 | 3.48/3.96 | 73.3 |
| 12 | 3.27 | 49.6 |
| 13 | 2.60/2.76 | 42.8 |

### Solvant DMSO

### Synthèse de 2,4,6-triméthyl-3-(oxiran-2-ylméthoxy)benzonitrile oxyde (D-1) :

A une solution de 2,4,6-triméthyl-3-(oxiran-2-ylméthoxy)benzaldéhyde oxime (17,00 g, 0,072 mol) dans du dichlorométhane (350 ml) refroidie jusqu'à 3°C est ajoutée au goutte à goutte une solution aqueuse de NaOCI dans l'eau (62,9 g Cl actif/L) (126 ml) pendant 10-15 minutes. La température du milieu réactionnel reste comprise entre 3 et 5°C. Le milieu réactionnel est ensuite agité pendant 1 heure à température 3-5°C. La phase aqueuse est séparée et extraite avec du dichlorométhane (25 ml). Les phases organiques réunies sont lavées avec de l'eau (3 fois 75 ml). Le solvant est évaporé à pression réduite (T_{bain} 22°C, 35 mbar). A ce résidu est additionné de l'éther de pétrole (40/60) (90 ml) et la suspension est maintenue sous agitation à température ambiante pendant 10-12 heures. Le précipité est filtré et lavé sur le filtre avec de l'éther de pétrole (3 fois par 30 ml) et enfin séché pendant 10-15 heures sous pression atmosphérique à température ambiante. Un solide blanc (15,12 g, rendement massique de 90 %) avec un point de fusion de 63°C est obtenu. La pureté molaire est supérieure à 99 % (RMN ¹H).

**Tableau d'attribution :**

| 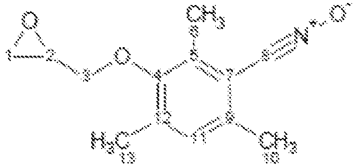 | | |
|---|---|---|
| | δ ¹H (ppm) | δ ¹³C (ppm) |
| 1 | 2.59/2.76 | 43.0 |
| 2 | 3.28 | 49.6 |
| 3 | 3.51/4.03 | 73.5 |
| 4 | / | 153.0 |
| 5 | / | 136.3 |
| 6 | 2.27 | 14.3 |
| 7 | / | 111.7 |
| 8 | / | / |
| 9 | / | 134.4 |
| 10 | 2.18 | 15.9 |
| 11 | 7.01 | 129.9 |
| 12 | / | 134.0 |
| 13 | 2.27 | 19.5 |

### Solvant DMSO

### Synthèse de 2,4,6-triméthyl-3-(3-(3,3-diméthyloxiran-2-yl)propoxy)benzonitrile oxyde (D-2):

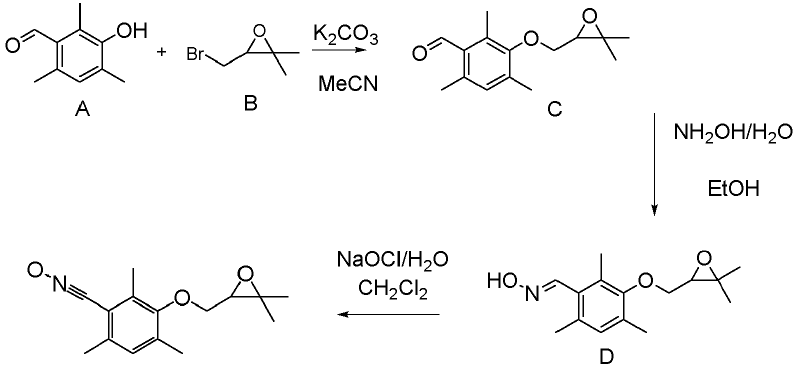

### Synthèse de 3-(bromométhyl)-2,2-diméthyloxirane (B) :

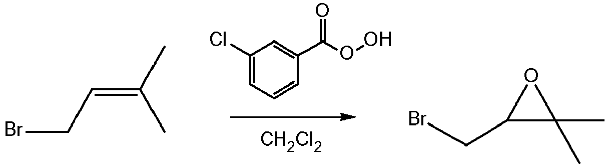

Le composé B peut être obtenu selon une procédure décrite dans l'article Shimizu, Hitoshi et al; Organic Process Research &Development, 9(3), 278-287; 2005

### Synthèse de 3-((3,3-diméthyloxiran-2-yl)méthoxy)-2,4,6-triméthylbenzaldéhyde (C) :

A un mélange de 3-hydroxy-2,4,6-triméthylbenzaldéhyde (19,20 g, 0,117 mol) et de 3-(bromométhyl)-2,2-diméthyloxirane (19,30 g, 0,117 mol) dans l'acétonitrile (50 ml) est ajouté le carbonate de potassium (12,12 g, 0,877 mol). Le milieu réactionnel est agité pendant 10-11 heures à 60 ºC (T_{bain}). Après retour à température ambiante, le mélange réactionnel est dilué avec un mélange d'eau (700 ml) et d'acétate d'éthyle (100 ml) et agité pendant 10 minutes. La phase aqueuse est séparée et extraite avec de l'acétate d'éthyle (3 fois 75 ml). Les phases organiques réunies sont lavées 2 fois avec une solution de NaOH (8,0 g dans 100 ml d'eau) et de l'eau (5 fois 75 ml). Le solvant est évaporé sous pression réduite (T_{bain} 35 ºC, 10 mbar). Une huile jaune claire (28,18 g, rendement massique de 97 %) est obtenue. La pureté molaire est supérieure à 85 % (RMN ¹H). Le produit C est utilisé pour l'étape suivante sans aucune purification supplémentaire.

**Tableau d'attribution :**

| | | |
|---|---|---|
| 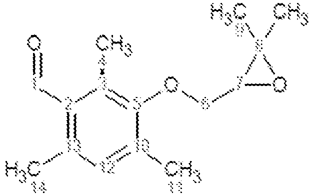 | | |

| | δ ¹H (ppm) | δ ¹³C (ppm) |
|---|---|---|
| 1 | 10.4 | 192.6 |
| 2 | / | 131.2 |
| 3 | / | 133.3 |
| 4 | 2.43 | 12 |
| 5 | / | 153.7 |
| 6 | 3.67 et3.87 | 71.3 |
| 7 | 3.09 | 61.1 |
| 8 | / | 57.8 |
| 9 | 1.17 et 1.28 | 18.6 et 24.3 |
| 10 | / | 125.8 |
| 11 | 2.21 | 16.5 |
| 12 | 6.79 | 13.5 |
| 13 | / | 136.4 |
| 14 | 2.4 | 19.5 |

### Solvant CDCl₃

### Synthèse de 3-((3,3-diméthyloxiran-2-yl)méthoxy)-2,4,6-triméthylbenzaldéhyde oxime (D) :

A une solution de 3-((3,3-diméthyloxiran-2-yl)méthoxy)-2,4,6-triméthylbenzaldéhyde (11,8 g, 0,475 mol) dans l'alcool éthylique (25 ml) est ajoutée à 40° C (T_{bain}) une solution d'hydroxylamine (5,02 g, 0,760 mol, 50 % dans l'eau, Aldrich) dans l'alcool éthylique (10 ml). Le milieu réactionnel est agité pendant 2,5-3,0 heures à température 55°C (T_{bain}). Après évaporation du solvant (T_{bain} 32°C, 26 mbar), un mélange d'acétate d'éthyle (20 ml), d'éther de pétrole (40/60) (30 ml) et d'eau (10 ml) est ajouté.

Ensuite, la phase organique est séparée et lavée avec de l'eau (10 ml). La solution est filtrée sur une couche de silicagel (Ø 3,5 cm, h = 2,0 cm), puis la couche de silicagel est lavée par un mélange d'acétate d'éthyle (10 ml) et d'éther de pétrole (20 ml). Après évaporation des solvants (T_{bain} 33°C, 11 mbar) une huile incolore (10,33 g, rendement massique de 83%) est obtenue. La pureté molaire est supérieure à 78 % (RMN ¹H) et 16% d'EtOAc. Le produit D est utilisé dans l'étape suivante sans séchage supplémentaire.

**Tableau d'attribution :**

| 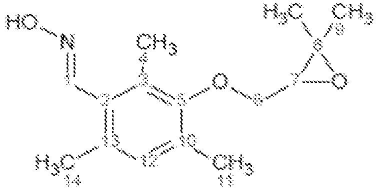 | | |
|---|---|---|
| | δ¹H (ppm) | δ ¹³C (ppm) |
| 1 | 8.29 | 149.2 |
| 2 | / | 128.3 |
| 3 | / | 129.9 |
| 4 | 2.27 | 13.3 |
| 5 | / | 153.5 |
| 6 | 3.76 et 3.88 | 71.2 |
| 7 | 3.15 | 61.4 |
| 8 | / | 58 |
| 9 | 1.22 et 1.33 | 18.6 et 24.4 |
| 10 | / | 131.3 |
| 11 | 2.22 | 16.1 |
| 12 | 6.83 | 130.5 |
| 13 | / | 132.7 |
| 14 | 2.25 | 20.4 |

### Solvant CDCl₃

### Synthèse du 3-((3,3-diméthyloxiran-2-yl)méthoxy)-2,4,6-triméthylbenzonitrile oxyde (D-2) :

A une solution de 3-((3,3-diméthyloxiran-2-yl)méthoxy)-2,4,6-trimethylbenzaldehyde oxime (9,90 g, 0.367 mol) dans le dichlorométhane (350 ml) refroidie jusqu'à 1-3°C est ajoutée au goutte à goutte une solution aqueuse de NaOCI dans l'eau (62,9 g Cl/L) (65 ml) pendant 15 minutes. La température du milieu réactionnel reste comprise entre 2-3°C. Le milieu réactionnel est ensuite agité pendant 2 heures à 2-3°C. La phase organique est séparée et lavée avec de l'eau (3 fois 50 ml). Le solvant est évaporé à pression réduite (T_{bain} 21°C, 120 mbar). A ce résidu est additionné de l'éther de pétrole (40/60) (15 ml) et la suspension est maintenue à -18ºC pendant 2 heures. Le précipité est filtré et lavé sur le filtre par l'éther de pétrole (3 fois par 15 ml) et enfin séché pendant 10-15 heures sous pression atmosphérique à température ambiante. Un solide blanc (4,42 g, rendement massique de 45 %) avec un point de fusion de 84°C est obtenu. La pureté molaire est supérieure à 98 % (RMN ¹H).

**Tableau d'attribution :**

| 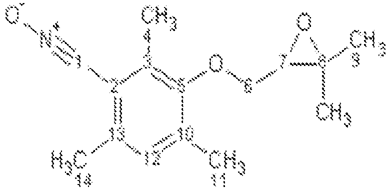 | | |
|---|---|---|
| | δ ¹H (ppm) | δ ¹³C (ppm) |
| 1 | / | / |
| 2 | / | 112.7 |
| 3 | / | 134.2 |
| 4 | 2.35 | 14.8 |
| 5 | / | 153.4 |
| 6 | 3.93/3.71 | 71.9 |
| 7 | 3.11 | 61.2 |
| 8 | / | 57.8 |
| 9 | 1.22 et 1.33 | 24.5/18.8 |
| 10 | / | 134.2 |
| 11 | 2.23 | 16.5 |
| 12 | 6.86 | 130.2 |
| 13 | / | 137.2 |
| 14 | 2.32 | 20 |

### Solvant CDCl₃

### Synthèse de 2,4,6-triméthyl-3-(3-(3-méthyloxiran-2-yl)propoxy)benzonitrile oxyde (D-3) :

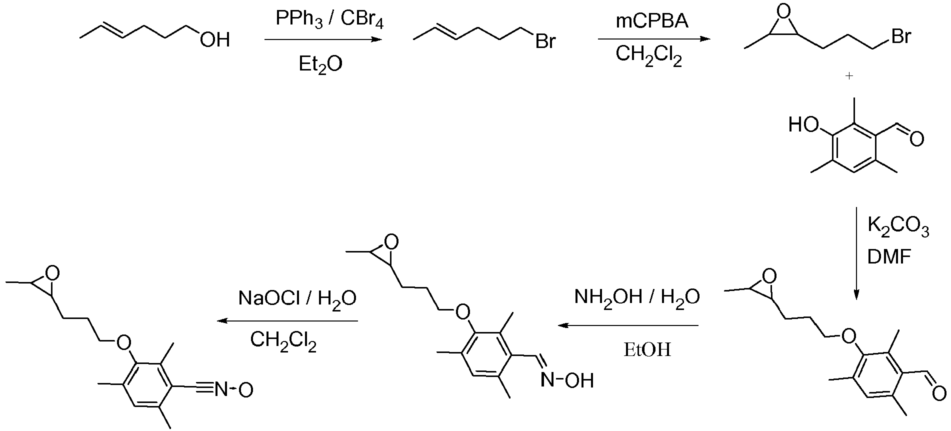

### Synthèse de 6-bromohex-2-ène :

Ce composé peut être obtenu par exemple selon une procédure décrite dans l'article Nicolai, Stefano et al. Tetrahedron, 71(35), 5959-5964; 2015

### Synthèse de 2-(3-bromopropyl)-3-méthyloxirane :

Ce composé peut être obtenu par exemple selon une procédure décrite dans l'article Hu, Shanghai; Hager, Lowell P.; Tetrahedron Letters; vol. 40; nb. 9; (1999); p. 1641 - 1644

### Synthèse du 2,4,6-triméthyl-3-(3-(3-méthyloxiran-2-yl)propoxy)benzaldéhyde :

A un mélange de 3-hydroxy-2,4,6-triméthylbenzaldéhyde (10,00 g, 0,061 mol) et de 2-(3-bromopropyl)-3-méthyloxirane (10,39 g, 0,058 mol) dans le DMF (5 ml) est ajouté le carbonate de potassium (6,01 g, 0,044 mol). Le milieu réactionnel est agité pendant 1 heure à 80 ºC (T_{bain}) et 3 heures à 100 ºC (T_{bain}). Après retour à température ambiante, le mélange réactionnel est dilué avec un mélange d'eau (75 ml) et de chlorure de méthylène (50 ml). Le produit est extrait avec le chlorure de méthylène (2 fois 10 ml). Les phases organiques réunies sont lavées 2 fois avec une solution de NaOH (4 g dans 50 ml d'eau) et de l'eau (3 fois par 15 ml). Le solvant est évaporé sous pression réduite (T_{bain} 45 ºC, 8 mbar). Une huile (14,25 g, rendement massique de 93 %) est obtenue. La pureté molaire est supérieure à 85 % (RMN ¹H). Le produit est utilisé pour l'étape suivante sans purification supplémentaire.

**Tableau d'attribution :**

| 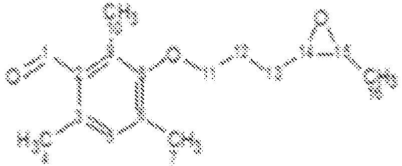 | | |
|---|---|---|
| | δ ¹H (ppm) | δ ¹³C (ppm) |
| 1 | 10.46 | 193.2 |
| 2 | / | 131.7 |
| 3 | / | 133.8 |
| 4 | 2.45 | 19.9 |
| 5 | 6.83 | 131.9 |
| 6 | / | 137 |
| 7 | 2.22 | 16.8 |
| 8 | / | 154.4 |
| 9 | / | 136.5 |
| 10 | 2.44 | 12.3 |
| 11 | 3.67 | 72.2 |
| 12 | 1.89 | 26.7 |
| 13 | 1.62-1.79 | 28.7 |
| 14 | 2.66 | 59.3 |
| 15 | 2.75 | 54.5 |
| 16 | 1.25 | 17.6 |

### Solvant CDCl₃

### Synthèse de 2,4,6-triméthyl-3-(3-(3-méthyloxiran-2-yl)propoxy)benzaldéhyde oxime :

A une solution de 2,4,6-triméthyl-3-(3-(3-methyloxiran-2-yl)propoxy)benzaldéhyde (14,00 g, 0,056 mol) dans l'alcool éthylique (40 ml) est ajoutée à 45°C une solution d'hydroxylamine (5,13 g, 0,078 mol, 50 % dans l'eau, Aldrich) dans l'alcool éthylique (10 ml). Le milieu réactionnel est agité pendant 1,5 heure à 50°C (T_{bain}). Après l'évaporation du solvant (T_{bain} 40°C, 45 mbar), du chlorure de méthylène (50 ml) est ajouté et la solution est lavée avec de l'eau (3 fois 15 ml). Ensuite, après évaporation du solvant (T_{bain} 40°C, 70 mbar), du chlorure de méthylène est ajouté. La suspension est agitée à température ambiante pendant 10 minutes et refroidie jusqu'à -18°C pendant 10-15 minutes. Le précipité est filtré et lavé sur le filtre 3 fois avec un mélange de chlorure de méthylène (1 ml) et d'éther de pétrole (4 ml) et enfin séché sous pression atmosphérique à température ambiante. Un solide blanc (10,02 g, rendement massique de 65 %) avec un point de fusion de 78°C est obtenu. La pureté molaire est supérieure à 90 % (RMN ¹H).
Tableau d'attribution :

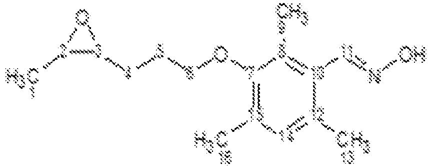

| | δ ¹H (ppm) | δ ¹³C (ppm) |
|---|---|---|
| 1 | 1.26 | 17.6 |
| 2 | 2.76 | 54.7 |
| 3 | 2.68 | 59.5 |
| 4 | 1.65/1.79 | 28.8 |
| 5 | 1.88 | 26.8 |
| 6 | 3.68 | 72.0 |
| 7 | / | 154.1 |
| 8 | / | 130.4 |
| 9 | 2.24 | 13.6 |
| 10 | / | 128.4 |
| 11 | 8.30 | 149.9 |
| 12 | / | 132.7 |
| 13 | 2.25 | 20.5 |
| 14 | 6.82 | 130.8 |
| 15 | / | 131.8 |
| 16 | 2.19 | 16.3 |

### Solvant CDCl₃

### Synthèse de 2,4,6-triméthyl-3-(3-(3-méthyloxiran-2-yl)propoxy)benzonitrile oxyde (D-3) :

A une solution de 2,4,6-triméthyl-3-(3-(3-méthyloxiran-2-yl)propoxy)benzaldéhyde oxime (3,35 g, 0,012 mol) dans le dichlorométhane (50 ml) refroidi jusqu'à 0°C (T_{bain}) est ajoutée au goutte à goutte une solution aqueuse de NaOCI dans l'eau (4% du chlore actif, Aldrich) (17 ml) pendant 5 minutes. La température du milieu réactionnel reste comprise entre 3 et 5°C. Le milieu réactionnel est ensuite agité pendant 1 heure à température 3-5°C. La phase aqueuse est séparée puis extraite avec du dichlorométhane (5 ml). Les phases organiques réunies sont lavées avec de l'eau (2 fois 5 ml). Le solvant est évaporé à pression réduite (T_{bain} 21°C, 16 mbar). A ce résidu est ajouté de l'éther de pétrole (40/60) (7 ml) et la suspension est agitée à température ambiante pendant 10 minutes. Le précipité est filtré et lavé sur le filtre par de l'éther de pétrole (2 fois 5 ml) et enfin séché sous pression atmosphérique à température ambiante. Un solide jaune clair (2,49 g, rendement massique de 75%) avec un point de fusion de 56°C est obtenu. La pureté molaire est supérieure à 94 % (RMN ¹H).
Tableau d'attribution :

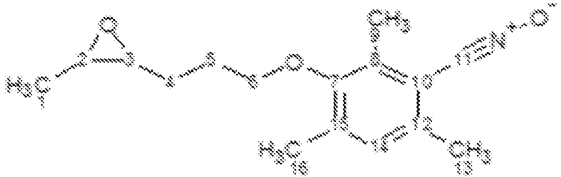

| | δ ¹H (ppm) | δ ¹³C (ppm) |
|---|---|---|
| 1 | 1.26 | 17.6 |
| 2 | 2.75 | 54.5 |
| 3 | 2.66 | 59.3 |
| 4 | 1.60/1.80 | 28.7 |
| 5 | 1.88 | 26.8 |
| 6 | 3.68 | 72.2 |
| 7 | / | 153.9 |
| 8 | / | 137.1 ou 134.6 |
| 9 | 2.31 | 14.9 |
| 10 | / | 112.8 |
| 11 | / | / |
| 12 | / | 137.1 ou 134.6 |
| 13 | 2.31 | 20.3 |
| 14 | 6.84 | 130.3 |
| 15 | / | 134.6 |
| 16 | 2.19 | 16.5 |

### Solvant CDCl₃

### Synthèse de 2,4,6-triméthyl-3-((3-phényloxiran-2-yl)méthoxy)benzonitrile oxyde (D-4) :

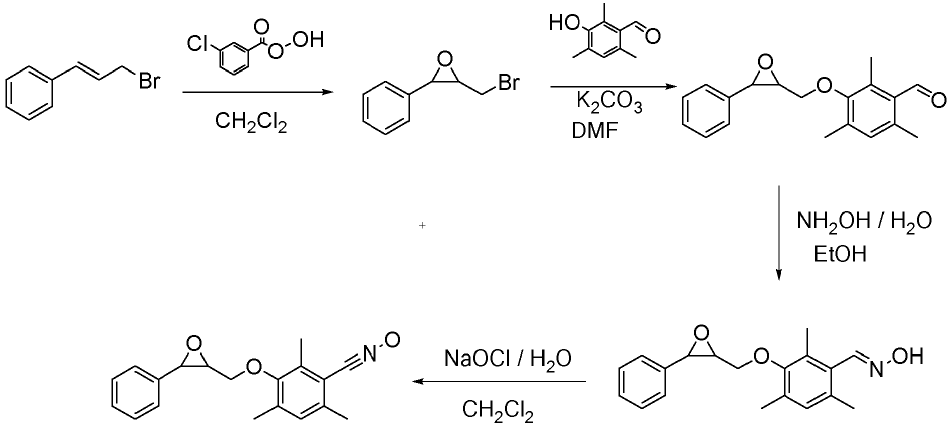

### Synthèse de 2-(bromométhyl)-3-phényloxirane :

Ce composé peut être obtenu selon une procédure décrite dans l'article Dickinson, Julia M. et al, Chemical Society, Perkin Transactions 1: Organic and Bio-Organic Chemistry (1972-1999), (4), 1179-84; 1990*.*

### Synthèse de 2,4,6-trimethyl-3-((3-phenyloxiran-2-yl)methoxy)benzaldehyde :

A un mélange de 3-hydroxy-2,4,6-triméthylbenzaldéhyde (13,50 g, 0,082 mol) et de 2-(bromométhyl)-3-phényloxirane (17,50 g, 0,082 mol) dans le DMF (8 ml) est ajouté le carbonate de potassium (8,51 g, 0,062 mol). Le milieu réactionnel est agité pendant 5-6 heures à 60 ºC (T_{bain}). Après retour à 40-50 ºC le mélange réactionnel est dilué avec un mélange d'eau (200 ml) et d'éthylacétate (70-80 ml). Le produit cible est extrait par avec de l'éthylacétate (2 fois 25 ml). Les phases organiques réunies sont lavées avec une solution de NaOH (8 g dans 70 ml d'eau) et d'eau (4 fois 25 ml). Le solvant est évaporé sous pression réduite (T_{bain} 34 ºC, 16 mbar). De l'éther de pétrole (40/60) (50 ml) est ajouté et le précipité est filtré et lavé sur le filtre avec un mélange d'éther de pétrole (15 ml) et d'éthylacétate (1 ml) et enfin séché sous pression atmosphérique à température ambiante.

Un solide beige (13,28 g, rendement massique de 55 %) avec un point de fusion de 53°C est obtenu. La pureté molaire est supérieure à 90 % (RMN ¹H).
Tableau d'attribution :

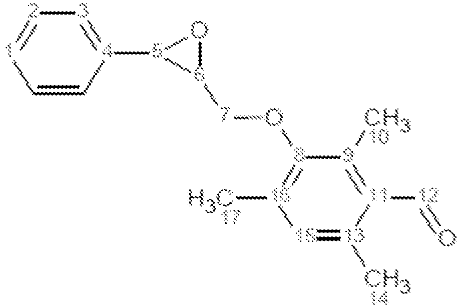

| | δ ¹H (ppm) | δ ¹³C (ppm) |
|---|---|---|
| 1/2 | 7.18 à 7.34 | 128.2/128.3 |
| 3 | | 125.5 |
| 4 | / | 136.2 |
| 5 | 3.81 | 55.9 |
| 6 | 3.35 | 60.2 |
| 7 | 3.84 et 4.04 | 72.5 |
| 8 | / | 153.8 |
| 9/13/16 | / | 132/133.7/136.7 |
| 10 | 2.5 | 12.2 |
| 11 | / | 131.5 |
| 12 | 10.48 | 193 |
| 14 | 2.48 | 19.8 |
| 15 | 6.87 | 131.8 |
| 17 | 2.28 | 16.6 |

### Solvant CDCl₃

### Synthèse de 2,4,6-triméthyl-3-((3-phényloxiran-2-yl)méthoxy)benzaldéhyde oxime :

A une solution de 2,4,6-triméthyl-3-((3-phényloxiran-2-yl)méthoxy)benzaldéhyde (4,60 g, 0,016 mol) dans de l'alcool éthylique (20 ml) est ajoutée à 45°C une solution d'hydroxylamine (1,43 g, 0,022 mol, 50 % dans l'eau, Aldrich) dans l'alcool éthylique (5 ml). Le milieu réactionnel est agité pendant 1,5 heure à 50°C (T_{bain}). Après retour à température ambiante, de l'eau (3 ml) est ajoutée à la suspension et la suspension est maintenue à -18ºC pendant 2 heures. Le précipité est filtré et lavé sur le filtre par un mélange d'alcool éthylique et d'eau (3 ml / 2 ml et 1 ml / 4 ml) et enfin séché sous pression atmosphérique à température ambiante. Un solide blanc (3,62 g, rendement massique de 75 %) avec un point de fusion de 125°C est obtenu. La pureté molaire est supérieure à 97 % (RMN ¹H).
Tableau d'attribution :

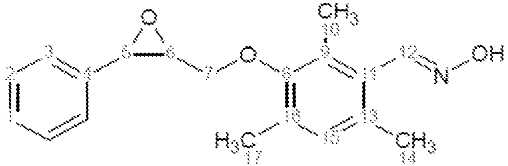

| | δ ¹H (ppm) | δ ¹³C (ppm) |
|---|---|---|
| 1/2 | 7.21 à 7.35 | 128/128.2 |
| 3 | | 125.4 |
| 4 | / | 136.2 |
| 5 | 3.81 | 56 |
| 6 | 3.35 | 60.3 |
| 7 | 3.85 et 4.02 | 72.2 |
| 8 | / | 153.4 |
| 9 | / | 130.2 |
| 10 | 2.29 | 13.1 |
| 11 | / | 128.2 |
| 12 | 8.31 | 149.6 |
| 13 | / | 132.9 |
| 14 | 2.27 | 20.3 |
| 15 | 6.85 | 130.7 |
| 16 | / | 131 |
| 17 | 2.24 | 16 |

### Solvant CDCl₃

### Synthèse de 2,4,6-triméthyl-3-((3-phényloxiran-2-yl)méthoxy)benzonitrile oxyde (D-4) :

A une solution de 2,4,6-triméthyl-3-((3-phényloxiran-2-yl)méthoxy)benzaldéhyde oxime (10,20 g, 0,033 mol) dans le dichlorométhane (150 ml) refroidi jusqu'à 4°C est ajoutée au goutte à goutte une solution aqueuse de NaOCI dans l'eau (74,4 g Cl/L) (48 ml) pendant 15 minutes. La température du milieu réactionnel reste comprise entre 3 et 5°C. Le milieu réactionnel est ensuite agité pendant 2,5 heures à température 3-5°C. La phase aqueuse est séparée et extraite avec du dichlorométhane (15 ml). Les solutions organiques réunies sont lavées avec de l'eau (3 fois 20 ml). Le solvant est évaporé à pression réduite (T_{bain} 23°C, 22mbar). De l'éther de pétrole (40/60) (60 ml) est ajouté et la suspension est agitée à température ambiante pendant 10-15 minutes. Le précipité est filtré et lavé sur le filtre avec de l'éther de pétrole (2 fois par 20 ml) et enfin séché sous pression atmosphérique à température ambiante. Un solide blanc (8,35 g, rendement massique de 82 %) avec un point de fusion de 64°C est obtenu. La pureté molaire est supérieure à 98 % (RMN ¹H).
Tableau d'attribution :

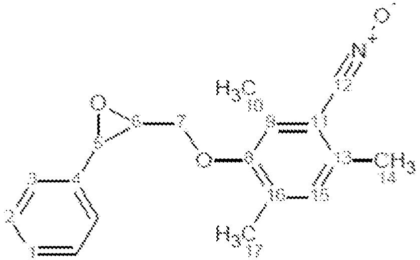

| | δ ¹H (ppm) | δ ¹³C (ppm) |
|---|---|---|
| 1/2 | 7.21 à 7.35 | 128.2/128.4 |
| 3 | | 125.4 |
| 4 | / | 136.1 |
| 5 | 3.79 | 55.8 |
| 6 | 3.33 | 60.1 |
| 7 | 3.82 et 4.05 | 72.5 |
| 8 | / | 153.3 |
| 9/13/16 | / | 130.3/134.4/ 137.3 |
| 10 | 2.36 | 14.6 |
| 11 | / | 112.8 |
| 12 | / | / |
| 14 | 2.33 | 20.1 |
| 15 | 6.87 | 130.2 |
| 17 | 2.25 | 16.4 |

### Solvant CDCl₃

### II.3-Préparation des compositions de caoutchouc :

On prépare cinq compositions de caoutchouc, respectivement T, C-1, C-2, C-3 et C-4. La formulation des compositions figure dans le tableau I.

La composition T est une composition témoin, puisqu'elle n'est pas à base de composé 1,3-dipolaire. La composition C-n (n allant de 1 à 4) est à base de composé dipolaire D-n.

La composition C-1 est une composition comparative, puisque le composé 1,3-dipolaire utilisé a un cycle époxyde dont un premier membre est un atome de carbone présentant un rattachement au dipôle, mais dont un deuxième membre n'est ni un carbone tertiaire, ni un carbone quaternaire.

Les compositions C-2 à C-4 sont conformes à l'invention, puisque chacun des composés 1,3-dipolaires utilisés ont un cycle époxyde dont un premier membre est un atome de carbone présentant un rattachement au dipôle et un deuxième membre est un carbone tertiaire (C-3 et C-4) ou un carbone quaternaire (C-2).

Les compositions de caoutchouc sont préparées selon le mode opératoire suivant :
Dans un mélangeur interne, dont la température de cuve initiale est d'environ 80°C, on introduit l'élastomère, le cas échéant le composé 1,3-dipolaire (0.5% molaire) et on malaxe l'ensemble pendant environ une minute. L'élastomère est un copolymère de 1,3-butadiène et de styrène à 26% d'unité styrène et à 56% d'unité butadiène-1,2 (vinyle). Ensuite, on introduit la charge renforçante, le silane, puis, après une à deux minutes de malaxage, les divers autres ingrédients à l'exception du système de vulcanisation. On conduit alors un travail thermomécanique (phase non-productive) en une étape (durée totale du malaxage égale à environ 5 minutes), jusqu'à atteindre une température maximale de "tombée" de 145°C. On récupère le mélange ainsi obtenu, on le refroidit puis on ajoute le système de vulcanisation (soufre) sur un mélangeur externe (homo-finisseur) à 25°C, en mélangeant le tout (phase productive) pendant environ 5 à 6 minutes. Le mélange est ensuite calandré sous forme de plaques (épaisseur de 2 à 3mm) pour la mesure des propriétés de traction et des propriétés dynamiques. Le mélange est ensuite vulcanisé, ses propriétés rhéométriques et ses propriétés à cuit sont mesurées.

Les résultats figurent dans le tableau II. Les résultats sont indiqués en base 100 par rapport à la composition témoin (T) : la valeur indiquée pour une composition est le rapport entre la valeur mesurée sur la composition et la valeur mesurée sur la composition témoin.

Les compositions C-2 à C-4 vulcanisées présentent un allongement à la rupture et une contrainte à la rupture améliorées par rapport à la composition C-1. Ces résultats sont obtenus sans être au détriment des propriétés d'hystérèse, puisque les valeurs de ΔG^{∗} et de Tan(δ) max restent très inférieures à celle de la composition témoin (T). Les valeurs de ΔC qui sont plus faibles que celle de la composition T corroborent une amélioration de l'interaction entre l'élastomère et la charge renforçante.

On observe aussi que les valeurs de Cmin des compositions C-2 à C-4 sont inférieures à celle de la composition C-1, ce qui indique une diminution de la viscosité à cru (avant vulcanisation) des compositions et augure une mise en œuvre au moins aussi aisée des compositions C-2 à C-4 que la composition T. Ce résultat est d'autant plus surprenant qu'on a constaté par ailleurs une amélioration de l'interaction entre l'élastomère et la charge renforçante.

En résumé, comparativement aux compositions non conformes à l'invention, les compositions de caoutchouc conformes à l'invention sont caractérisées par un compromis amélioré entre les propriétés rupture, les propriétés d'hystérèse et les propriétés de mise en œuvre.

**Tableau I**

| Composition | T | C-1 | C-2 | C-3 | C-4 |
|---|---|---|---|---|---|
| SBR | 100 | | | | |
| SBR + D-1 | | 100 | | | |
| SBR +D-2 | | | 100 | | |
| SBR + D-3 | | | | 100 | |
| SBR + D-4 | | | | | 100 |
| Silice (1) | 60 | 60 | 60 | 60 | 60 |
| Silane (2) | 4,8 | 4,8 | 4,8 | 4,8 | 4,8 |
| Antioxydant (3) | 3 | 3 | 3 | 3 | 3 |
| Paraffine (4) | 1 | 1 | 1 | 1 | 1 |
| ZnO (5) | 2,7 | 2,7 | 2,7 | 2,7 | 2,7 |
| Acide stéarique | 2,5 | 2,5 | 2,5 | 2,5 | 2,5 |
| CBS (6) | 1,8 | 1,8 | 1,8 | 1,8 | 1,8 |
| Soufre | 1,5 | 1,5 | 1,5 | 1,5 | 1,5 |

| | | | | | |
|---|---|---|---|---|---|
| (1) Silice « 160 MP » commercialisée par Solvay (2) TESPT commercialisé par Evonik sous la référence « SI69» (3) N-(l,3-diméthylbutyl)-N'-phényl-p-phénylènediamine de la société Flexsys (4) Agent de mise en œuvre Paraffine 6266 (5) Oxyde de zinc (6) N-cylohexyl-2-benzothiazol-sulfénamide (« Santocure » de la société Flexsys) | | | | | |

**Tableau II**

| Composition | T | C-1 | C-2 | C-3 | C-4 |
|---|---|---|---|---|---|
| Allongement rupture | 100 | 58 | 84 | 68 | 74 |
| Contrainte rupture | 100 | 90 | 111 | 97 | 97 |
| ΔG^{∗} | 100 | 71 | 79 | 88 | 88 |
| Tan(δ) max | 100 | 73 | 73 | 81 | 73 |
| C min | 100 | 110 | 74 | 74 | 84 |
| ΔC | 100 | 68 | 76 | 94 | 66 |

## Revendications

1. Composition de caoutchouc à base d'au moins un élastomère diénique, une charge renforçante et un composé 1,3-dipolaire comportant un groupe époxyde, le groupe époxyde étant un cycle éther à 3 membres dans lequel un premier membre est un atome de carbone présentant un rattachement au dipôle du composé 1,3-dipolaire et un deuxième membre est un carbone tertiaire ou quaternaire.

2. Composition de caoutchouc selon la revendication 1 dans laquelle le groupe époxyde est de formule (I) 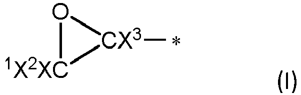 dans laquelle
- ^{∗} représente un rattachement au dipôle,
- X¹, X² et X³, identiques ou différents, représentent un atome d'hydrogène ou un groupe substituant, et
- au moins un des X¹, X² et X³ est différent d'un atome d'hydrogène.

3. Composition de caoutchouc selon la revendication 2 dans laquelle le groupe substituant est un alkyle ou un aryle.

4. Composition de caoutchouc selon l'une quelconque des revendications 1 à 3, dans laquelle le composé 1,3-dipolaire est choisi dans le groupe constitué par les oxydes de nitrile, les nitrones et les nitriles imine, de préférence un oxyde de nitrile.

5. Composition de caoutchouc selon l'une quelconque des revendications 1 à 4, dans laquelle le composé 1,3-dipolaire comprend un noyau benzénique substitué par le dipôle du composé 1,3-dipolaire.

6. Composition de caoutchouc selon la revendication 5 dans laquelle le noyau benzénique est substitué en ortho du dipôle.

7. Composition de caoutchouc selon l'une quelconque des revendications 1 à 6, dans laquelle le composé est un monooxyde de nitrile aromatique.

8. Composition de caoutchouc selon l'une quelconque des revendications 1 à 7, dans laquelle le composé contient un motif de formule (II) 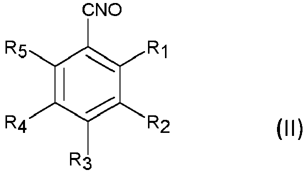 dans laquelle :
- quatre des cinq symboles R1 à R5, identiques ou différents, sont chacun un atome ou un groupe d'atomes, et le cinquième symbole représente une chaîne carbonée permettant le rattachement au groupe époxyde, sachant qu'au moins un des R1 et R5 est différent d'un atome d'hydrogène.

9. Composition de caoutchouc selon la revendication 8 dans laquelle R1, R3 et R5 représentent chacun un groupe hydrocarboné, de préférence alkyle, de manière plus préférentielle méthyle ou éthyle.

10. Composition de caoutchouc selon l'une quelconque des revendications 8 à 9 dans laquelle le cinquième symbole représente une chaîne carbonée interrompue par un ou plusieurs hétéroatomes, de préférence oxygène.

11. Composition de caoutchouc selon l'une quelconque des revendications 8 à 10 dans laquelle le cinquième symbole comprend un motif -CH₂O-, le groupe méthylène étant attaché au groupe époxyde.

12. Composition de caoutchouc selon l'une quelconque des revendications 1 à 11 dans laquelle le composé 1,3-dipolaire a pour formule l'une des formules (III), (IV) ou (V)

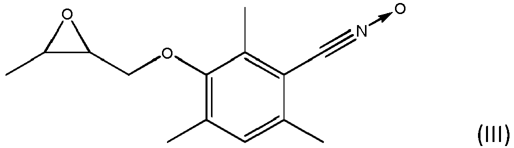

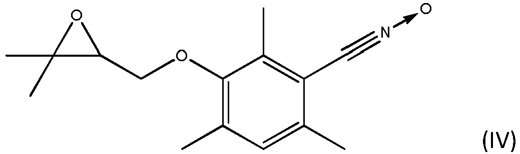

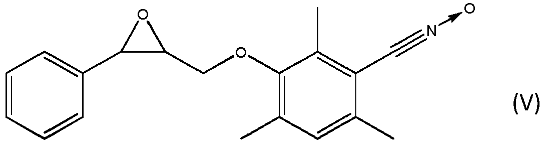

13. Composition de caoutchouc selon l'une quelconque des revendications 1 à 12 dans laquelle l'élastomère diénique est choisi dans le groupe des élastomères constitué par les homopolymères de 1,3-butadiène, les homopolymères d'isoprène, les copolymères de 1,3-butadiène, les copolymères d'isoprène et leurs mélanges.

14. Composition de caoutchouc selon l'une quelconque des revendications 1 à 13 dans laquelle la charge renforçante comprend une charge inorganique renforçante, de préférence une silice.

15. Pneumatique qui comprend une composition de caoutchouc définie à l'une quelconque des revendications 1 à 14.

## Patentansprüche

1. Kautschukzusammensetzung auf Basis von mindestens einem Dienelastomer, einem verstärkenden Füllstoff und einer 1,3-dipolaren Verbindung mit einer Epoxidgruppe, wobei es sich bei der Epoxidgruppe um einen 3-gliedrigen Etherring handelt, in dem ein erstes Glied ein Kohlenstoffatom mit einer Anbindung an den Dipol der 1,3-dipolaren Verbindung ist und ein zweites Glied ein tertiäres oder quaternäres Kohlenstoffatom ist.

2. Kautschukzusammensetzung nach Anspruch 1, wobei die Epoxidgruppe die Formel (I) aufweist: 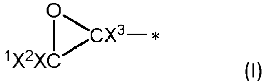 wobei
- * für eine Anbindung an den Dipol steht,
- X¹, X² und X³ gleich oder verschieden sind und für ein Wasserstoffatom oder eine Substituentengruppe stehen und
- mindestens eines von X¹, X² und X³ von einem Wasserstoffatom verschieden ist.

3. Kautschukzusammensetzung nach Anspruch 2, wobei es sich bei der Substituentengruppe um ein Alkyl oder ein Aryl handelt.

4. Kautschukzusammensetzung nach einem der Ansprüche 1 bis 3, wobei die 1,3-dipolaren Verbindung aus der Gruppe bestehend aus Nitriloxiden, Nitronen und Nitriliminen, vorzugsweise einem Nitriloxid, ausgewählt ist.

5. Kautschukzusammensetzung nach einem der Ansprüche 1 bis 4, wobei die 1,3-dipolare Verbindung einen durch den Dipol der 1,3-dipolaren Verbindung substituierten Benzolkern umfasst.

6. Kautschukzusammensetzung nach Anspruch 5, wobei der Benzolkern in ortho-Position zum Dipol substituiert ist.

7. Kautschukzusammensetzung nach einem der Ansprüche 1 bis 6, wobei es sich bei der Verbindung um ein aromatisches Nitrilmonooxid handelt.

8. Kautschukzusammensetzung nach einem der Ansprüche 1 bis 7, wobei die Verbindung eine Einheit der Formel (II) umfasst: 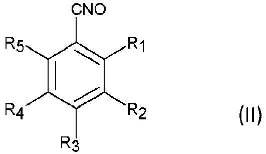 wobei:
- vier der fünf Symbole R₁ bis R₅ gleich oder verschieden sind und jeweils für ein Atom oder eine Gruppe von Atomen stehen und das fünfte Symbol für eine die Anbindung an die Epoxidgruppe ermöglichende Kohlenstoffkette steht, mit der Maßgabe, dass mindestens eines von R₁ bis R₅ von einem Wasserstoffatom verschieden ist.

9. Kautschukzusammensetzung nach Anspruch 8, wobei R₁, R₃ und R₅ jeweils für eine Kohlenwasserstoffgruppe, vorzugsweise eine Alkylgruppe, weiter bevorzugt eine Methyl- oder Ethylgruppe, stehen.

10. Kautschukzusammensetzung nach einem der Ansprüche 8 bis 9, wobei das fünfte Symbol für eine durch ein oder mehrere Heteroatome, vorzugsweise Sauerstoff, unterbrochene Kohlenstoffkette steht.

11. Kautschukzusammensetzung nach einem der Ansprüche 8 bis 10, wobei das fünfte Symbol eine -CH₂O-Einheit umfasst, wobei die Methylengruppe an die Epoxidgruppe gebunden ist.

12. Kautschukzusammensetzung nach einem der Ansprüche 1 bis 11, wobei die 1,3-dipolare Verbindung als Formel eine der Formeln (III), (IV) oder (V) aufweist:

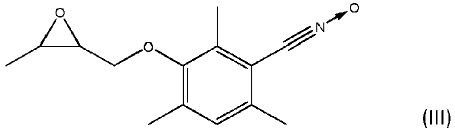

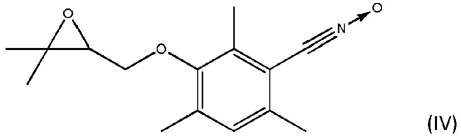

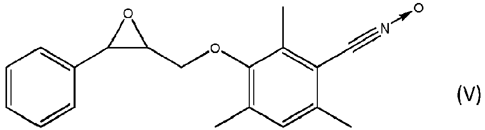

13. Kautschukzusammensetzung nach einem der Ansprüche 1 bis 12, wobei das Dienelastomer aus der Gruppe von Elastomeren bestehend aus 1,3-Butadien-Homopolymeren, Isopren-Homopolymeren, 1,3-Butadien-Copolymeren, Isopren-Copolymeren und Mischungen davon ausgewählt ist.

14. Kautschukzusammensetzung nach einem der Ansprüche 1 bis 13, wobei der verstärkende Füllstoff einen verstärkendem anorganischen Füllstoff, vorzugsweise eine Kieselsäure, umfasst.

15. Reifen, der eine Kautschukzusammensetzung gemäß einem der Ansprüche 1 bis 14 umfasst.

## Claims

1. Rubber composition based on at least one diene elastomer, a reinforcing filler and a 1,3-dipolar compound comprising an epoxide group, the epoxide group being a 3-membered ether ring in which a first member is a carbon atom exhibiting a connection to the dipole of the 1,3-dipolar compound and a second member is a tertiary or quaternary carbon.

2. Rubber composition according to Claim 1, in which the epoxide group is of formula (I): 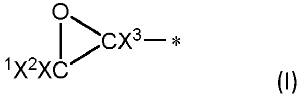 in which:
- ^{∗} represents a connection to the dipole,
- X¹, X² and X³, which are identical or different, represent a hydrogen atom or a substituent group, and
- at least one of X¹, X² and X³ is other than a hydrogen atom.

3. Rubber composition according to Claim 2, in which the substituent group is an alkyl or an aryl.

4. Rubber composition according to any one of Claims 1 to 3, in which the 1,3-dipolar compound is selected from the group consisting of nitrile oxides, nitrones and nitrilimines, preferably is a nitrile oxide.

5. Rubber composition according to any one of Claims 1 to 4, in which the 1,3-dipolar compound comprises a benzene nucleus substituted by the dipole of the 1,3-dipolar compound.

6. Rubber composition according to Claim 5, in which the benzene nucleus is substituted in the position ortho to the dipole.

7. Rubber composition according to any one of Claims 1 to 6, in which the compound is an aromatic nitrile monooxide.

8. Rubber composition according to any one of Claims 1 to 7, in which the compound contains a unit of formula (II): 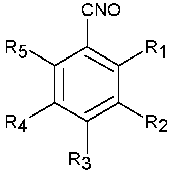 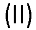 in which:
- four of the five symbols R₁ to R₅, which are identical or different, are each an atom or a group of atoms, and the fifth symbol represents a carbon chain which makes possible the connection to the epoxide group, it being known that at least one of R₁ and R₅ is other than a hydrogen atom.

9. Rubber composition according to Claim 8, in which R₁, R₃ and R₅ each represent a hydrocarbon group, preferably an alkyl group, more preferentially a methyl or ethyl group.

10. Rubber composition according to either one of Claims 8 and 9, in which the fifth symbol represents a carbon chain interrupted by one or more heteroatoms, preferably oxygen.

11. Rubber composition according to any one of Claims 8 to 10, in which the fifth symbol comprises a -CH₂O- unit, the methylene group being attached to the epoxide group.

12. Rubber composition according to any one of Claims 1 to 11, in which the 1,3-dipolar compound has, as formula, one of the formulae (III), (IV) or (V):

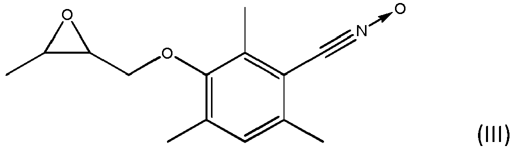

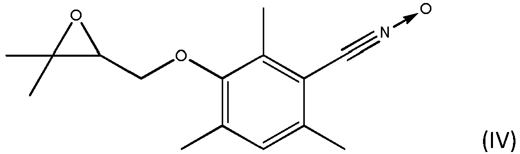

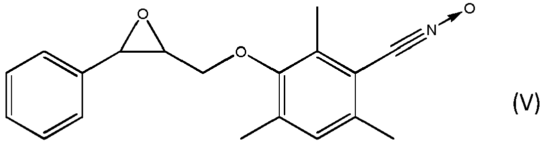

13. Rubber composition according to any one of Claims 1 to 12, in which the diene elastomer is selected from the group of elastomers consisting of 1,3-butadiene homopolymers, isoprene homopolymers, 1,3-butadiene copolymers, isoprene copolymers and their mixtures.

14. Rubber composition according to any one of Claims 1 to 13, in which the reinforcing filler comprises a reinforcing inorganic filler, preferably a silica.

15. Tyre which comprises a rubber composition defined in any one of Claims 1 to 14.
